# EUROPEAN PATENT APPLICATION

(11) **EP 0 592 875 A1**
(43) Date of publication of application: **20.04.1994**
(21) Application number: 93115782.0
(22) Date of filing: 30.09.1993
(51) Int. Cl.: C07C 315/00, C07C 317/14

(54) **Process for producing dichlorodiphenylsulfone**

(30) Priority: 06.10.1992 JP 267402/92; 30.07.1993 JP 190335/93
(71) Applicant: UBE INDUSTRIES, LTD., Ube-shi, Yamaguchi-ken 755 (JP)
(72) Inventor: Kashima, Mikito, c/o Chiba Laboratory, Ichihara-shi, Chiba (JP); Noda, Yumiki, c/o Chiba Laboratory, Ichihara-shi, Chiba (JP); Akiba, Yoshikazu, c/o Chiba Laboratory, Ichihara-shi, Chiba (JP)
(74) Representative: Hoeger, Stellrecht & Partner

(57) **Abstract**

Dichlorodiphenylsulfone is prepared with an enhanced yield by (1) dehydration-condensing chlorobenzene with 4-chlorobenzenesulfonic acid; (2) adding water to the resultant reaction product mixture in a 4-chlorobenzenesulfonic acid/water molar ratio of 1:0.8 to 1:2 to convert the non-reacted 4-chlorobenzenesulfonic acid to monohydrate thereof and allow the resultant monohydrate to precipitate; (3) recovering the precipitated 4-chlorobenzenesulfonic acid monohydrate; (4) collecting the reaction product consisting of dichlorodiphenylsulfone; and optionally (5) dehydrating the recovered 4-chlorobenzenesulfonic acid monohydrate and returning the dehydrated 4-chlorobenzenesulfonic acid to the dehydration-condensing step (1).

## Description

### BACKGROUND OF THE INVENTION

### 1. Field of the Invention

The present invention relates to a process for producing dichlorodiphenylsulfone. More particularly, the present invention relates to a process for continuously producing dichlorodiphenylsulfone at an enhanced yield and efficiency.

### 2. Description of Related Art

It is known to synthesize dichlorodiphenylsulfone by reacting chlorobenzene with 4-chlorobenzenesulfonic acid at elevated temperatures. This synthetic reaction is disadvantageous in that a complete conversion of 4-chlorobenzenesulfonic acid to dichlorodiphenylsulfone at a conversion of 100% is difficult and thus, usually, the resultant reaction product mixture contains a certain amount of non-reacted 4-chlorobenzenesulfonic acid mixed with the aimed reaction product consisting of dichlorodiphenylsulfone.

With respect to a conventional method of collecting dichlorodiphenylsulfone from the resultant reaction product mixture, for example, U. S. Patent No. 2,593,001 discloses a method of washing and removing the non-reacted 4-chlorobenzenesulfonic acid by adding a large amount of water to the reaction product mixture. This conventional method is disadvantageous in that the large amount of water must be removed from the resultant aqueous solution of the non-reacted 4-chlorobenzenesulfonic acid obtained by the water-washing step, to recover and re-use the non-reacted 4-chlorobenzenesulfonic acid. Therefore, this method cannot be utilized as an industrial and commercial method.

Also, International Publication No. WO 88/01617 discloses an improved process for preparing diarylsulfones. In this process, a limited amount of water is added to a reaction product mixture obtained by a reaction of a monosubstituted benzene (for example, chlorobenzene) with a monosubstituted benzene sulfonic acid (for example, 4-chlorobenzenesulfonic acid), to form a heavier aqueous phase containing unreacted monosubstituted benzene sulfonic acid and a lighter organic phase containing the resultant reaction product consisting of a diarylsulfone (for example, dichlorodiphenylsulfone), the heavier aqueous phase is separated from the lighter organic phase, and the aqueous phase is subjected to a dehydration procedure to recover and re-use the unreacted monosubstituted benzene sulfonic acid.

With respect to this process, it is emphasized that the amount of water contained in the recovered 4-chlorobenzenesulfonic acid is smaller than that obtained by the above-mentioned process in which a large amount of water is added to the reaction product mixture, and thus the dehydration of the recovered 4-chlorobenzenesulfonic acid is easy. This method comprises, however, as essential steps, an aqueous extraction step in which the non-reacted 4-chlorobenzenesulfonic acid is dissolved and extracted in water and a liquid phase-separation step in which the aqueous liquid phase is separated from the organic liquid phase. Also, in the first cycle of this process steps, the resultant aqueous phase contains about 4 to about 30% by weight of the resultant dichlorodiphenylsulfone together with the non-reacted 4-chlorobenzenesulfonic acid, and the organic phase contains about 3 to about 10% by weight of 4-chlorobenzenesulfonic acid together with the resultant dichlorodiphenylsulfone. Therefore, the resultant dichlorodiphenylsulfone must be collected not only from the organic phase but also the aqueous phase, and the non-reacted 4-chlorobenzenesulfonic acid must be recovered not only from the aqueous phase but also from the organic phase. For this purpose, the aqueous and organic phases must be further treated.

### SUMMARY OF THE INVENTION

An object of the present invention is to provide a process for producing dichlorodiphenylsulfone by a dehydration condensation reaction of chlorobenzene with 4-chlorobenzenesulfonic acid, with a high yield at a high efficiency.

Another object of the present invention is to provide a process for producing dichlorodiphenylsulfone by a dehydration condensation reaction of chlorobenzene with 4-chlorobenzenesulfonic acid, in which process, a non-reacted fraction of 4-chlorobenzenesulfonic acid is separated and recovered from the resultant reaction product mixture at a high efficiency, and the recovered 4-chlorobenzenesulfonic acids is re-used to produce an additional amount of dichlorodiphenylsulfone.

The above-mentioned object can be attained by the process of the present invention for producing dichlorodiphenylsulfone comprising the steps of:
(1) dehydration-condensing chlorobenzene with 4-chlorobenzenesulfonic acid while removing water generated as a by-product from the reaction mixture, to prepare dichlorodiphenylsulfone;
(2) adding water to the resultant reaction product mixture in a molar ratio of a non-reacted fraction of 4-chlorobenzenesulfonic acid to water of from 1:0.8 to 1:2, to convert the non-reacted 4-chlorobenzenesulfonic acid to a monohydrate thereof and to allow the resultant 4-chlorobenzenesulfonic acid monohydrate to precipitate from the reaction product mixture;
(3) recovering the precipitated 4-chlorobenzenesulfonic acid monohydrate from the reaction product mixture, to leave a residual reaction product solution; and
(4) collecting the reaction product consisting of dichlorodiphenylsulfone from the residual reaction product solution.

In the process of the present invention, the non-reacted 4-chlorobenzenesulfonic acid is recovered at a high efficiency, in the form of a monohydrate thereof and also, the aimed dichlorodiphenylsulfone is obtained with a high yield. In an embodiment of the process of the present invention, the recovered 4-chlorobenzenesulfonic acid monohydrate is dehydrated and then the resultant anhydrous 4-chlorobenzenesulfonic acid is returned into the dehydration-condensing step (1), to produce an additional amount of dichlorodiphenylsulfone.

In this embodiment, the recovered 4-chlorobenzenesulfonic acid monohydrate is easily converted to the original 4-chlorobenzenesulfonic acid at a high conversion and re-utilized to produce dichlorodiphenylsulfone.

### DESCRIPTION OF PREFERRED EMBODIMENTS

For the process of the present invention, 4-chlorobenzenesulfonic acid usable as a starting material, can be prepared by a well known conventional process in which chlorobenzene is reacted with a sulfonating agent, for example, fuming sulfuric acid, anhydrous sulfuric acid, concentrated sulfuric acid or chlorosulfonic acid.

In the process of the present invention, the first step (1) is carried out by a dehydration-condensation reaction of chlorobenzene with 4-chlorobenzenesulfonic acid while removing a by-product consisting of water from the reaction mixture to prepare dichlorodiphenylsulfone. This dehydration-condensation reaction is carried out by bringing chlorobenzene and 4-chlorobenzenesulfonic acid into contact with each other in the presence of a conventional condensation agent or catalyst, for example, P₂O₅ or PCℓ₅, or in the absence of the condensation agent or catalyst at a high temperature of, for example, from 180°C to 300°C, under the ambient atmospheric pressure or a raised pressure. The reaction causes a production of an aimed reaction product consisting of dichlorodiphenylsulfone and a by-product comprising water.

The dehydration-condensing step is carried out while removing water generated as a by-product from the reaction mixture.

The dehydration-condensation step (1) of the process of the present invention can be carried out by a batch method in which chlorobenzene and 4-chlorobenzenesulfonic acid are reacted batchwise with each other at a high temperature of, for example, from 180°C to 300°C, under a raised pressure, or by a semi-batch method in which chlorobenzene is continuously fed into a reaction system containing 4-chlorobenzenesulfonic acid to prepare dichlorodiphenylsulfone at a high temperature under a raised pressure, while continuously withdrawing an azeotropic mixture of chlorobenzene and the by-product consisting of water from the reaction system. In another method for producing dichlorodiphenylsulfone, a stream of chlorobenzene is continuously brought countercurrently or concurrently into contact with a stream of 4-chlorobenzenesulfonic acid under the ambient atmospheric pressure. This method is referred to as an ambient atmospheric pressure flow type method.

Among the above-mentioned methods, the batch and semi-batch methods are advantageous in that the dehydration-condensing procedure and the dehydration procedure for 4-chlorobenzenesulfonic acid monohydrate can be in one and the same reaction vessel.

The dehydration-condensation reaction of chlorobenzene with 4-chlorobenzenesulfonic acid is carried out preferably at a temperature of from 200°C to 290°C. The reaction pressure may be the ambient atmospheric pressure, a reduced pressure or a raised pressure. In view of a high reacting rate, the reaction is carried out preferably under a raised pressure.

As mentioned above, the condensation reaction of 4-chlorobenzenesulfonic acid with chlorobenzene generates water as a by-product. The water can be continuously removed as an azeotropic mixture with chlorobenzene from the reaction system by an azeotropic distillation at the temperature in the above-mentioned range.

In the second step (2) of the process of the present invention, a specific amount of water is added to the reaction product mixture of the dehydration condensation step (1). The specific amount of water is controlled to such an extent that a molar ratio of water to a non-reacted fraction of 4-chlorobenzenesulfonic acid in the reaction product mixture becomes 1:0.8 to 1:2, so that the non-reacted 4-chlorobenzenesulfonic acid is converted to a monohydrate thereof, and the resultant monohydrate of 4-chlorobenzenesulfonic acid is precipitated from the reaction product mixture.

Before the water-adding step (2), an amount of organic medium contained in the resultant reaction product mixture from the dehydration-condensation step (1), is preferably controlled to an appropriate level in consideration of a solubility of dichlorodiphenylsulfone in the organic medium. Namely, in the water-adding step (2), the reaction product mixture is preferably in the state of a solution in which the reaction product consisting of dichlorodiphenylsulfone is completely dissolved in an organic medium comprising the non-reacted chlorobenzene. If the amount of the non-reacted chlorobenzene contained in the reaction product mixture is not sufficiently large to completely dissolve therein the entire amount of the resultant dichlorodiphenylsulfone, an organic medium is preferably added in a certain amount thereof large enough to cause the resultant amount of organic medium to completely dissolve therein the resultant dichlorodiphenylsulfone.

If an excess amount of the organic medium is contained in the reaction product mixture, a portion of the organic medium may be evaporated away from the reaction product mixture. Preferably, the organic medium is present in a necessary and minimum amount for completely dissolving therein the resultant dichlorodiphenylsulfone. However, there is no limitation on the amount of the organic medium contained in the reaction product mixture. If the organic medium is present in too large an amount in the reaction product mixture, the collection efficiency of the resultant dichlorodiphenylsulfone becomes unsatisfactorily reduced.

The organic medium for dissolving therein the resultant dichlorodiphenylsulfone is not limited to chlorobenzene. Namely the organic medium for the solution of the reaction product mixture comprises a member selected from the group consisting of chlorobenzene, benzene, toluene, xylene, pseudcumene, tetrahydronaphthalene (tetralin), dichloromethane, 1,2-dichloroethane and mixtures of two or more of the above-mentioned compounds.

Particularly, since 4-chlorobenzenesulfonic acid exhibits a relatively low solubility in toluene and 1,2-dichloroethane, the reaction medium containing toluene and/or 1,2-dichloroethane exhibits a high separation performance for 4-chlorobenzenesulfonic acid. Where the above-mentioned organic medium other than chlorobenzene is used as the organic medium, the non-reacted chlorobenzene in the reaction product mixture is removed by distillation and then the organic medium is added in a sufficient amount to completely dissolve therein the resultant dichlorodiphenylsulfone. This amount of the organic medium is preferably a minimum amount necessary to completely dissolve therein the resultant dichlorodiphenylsulfone. However, the organic medium may be used in an amount greater than the minimum amount. In the water-adding step (2) of the process of the present invention, the amount of water added to the reaction product mixture is controlled so that the molar ratio of the non-reacted 4-chlorobenzenesulfonic acid contained in the reaction product mixture to water is in the range of from 1:0.8 to 1:2, preferably from 1:0.9 to 1:1.2. If the molar ratio is more than 1:0.8 (when water is used in too small an amount), the resultant 4-chlorobenzenesulfonic acid monohydrate is not precipitated to a satisfactory extent. Also, the molar ratio is less than 1:2 (when water is added in an excessively large amount), the precipitated 4-chlorobenzenesulfonic acid monohydrate deliquesces or becomes an aqueous solution thereof, and thus the resultant 4-chlorobenzenesulfonic acid monohydrate is recovered with a reduced recovery efficiency.

The conversion of anhydrous 4-chlorobenzenesulfonic acid to 4-chlorobenzenesulfonic acid monohydrate is carried out by an exothermic reaction, and thus the temperature of the reaction product mixture raises due to the exothermic reaction. Also, the solubility of 4-chlorobenzenesulfonic acid monohydrate in the organic medium is variable depending on the temperature of the organic medium. Namely, the higher the temperature of the organic medium, the higher the solubility of 4-chlorobenzenesulfonic acid monohydrate in the organic medium.

To recover the precipitated 4-chlorobenzenesulfonic acid monohydrate from the reaction product mixture by filtration at a high reproducibility, it is preferable that after the hydration reaction of 4-chlorobenzenesulfonic acid is completed, and that the resultant reaction product mixture is aged at a temperature sufficient for promoting the precipitation of 4-chlorobenzenesulfonic acid monohydrate, and is then subjected to the filtration procedure. Since the melting point of 4-chlorobenzenesulfonic acid monohydrate is 101°C, the recovering step (3) of the precipitated 4-chlorobenzenesulfonic acid monohydrate is carried out preferably at a temperature of lower than 101°C.

In consideration of collection efficiency and economy of 4-chlorobenzenesulfonic acid monohydrate, the recovering step (3) is carried out more preferably at a temperature of from 0°C to 70°C, usually at room temperature.

In the process of the present invention, the contents of the non-reacted chlorobenzenesulfonic acid and the reaction product consisting of dichlorodiphenylsulfone in the reaction product mixture obtained in the dehydration-condensing step (1) can be determined by sampling and analyzing a portion of the reaction product mixture after the dehydration-condensing step (1) is completed and before the water adding step (2) is started. There is no limitation on the analysis method. For example, a sample of the reaction product mixture has added thereto a certain amount of water to extract 4-chlorobenzenesulfonic acid in water and to separate a resultant aqueous phase from an organic phase. The amount of 4-chlorobenzenesulfonic acid extracted in the aqueous phase can be determined by an acid-base titration. Also, the contents of the resultant dichlorodiphenylsulfone and the non-reacted chlorobenzene is determined by concentrating the organic phase. Generally, sulfuric acid is contained in a negligibly small amount in the reaction product mixture. After the precipitated 4-chlorobenzenesulfonic acid monohydrate is recovered from the reaction product mixture by, for example, filtration, a solution containing dichlorodiphenylsulfone is left as a residue, for example, a filtrate.

This residual reaction product solution is subjected to the collecting step (4) for the aimed dichlorodiphenylsulfone. This collecting step (4) is carried out by subjecting the residual solution to a distillation procedure in which the organic medium is evaporated away. Then, the resultant dichlorodiphenylsulfone is collected as a distillation residue of the reaction product solution. In the collecting step (4) of the process of the present invention, the resultant dichlorodiphenylsulfone is collected.

In an embodiment of the process of the present invention, the recovered 4-chlorobenzenesulfonic acid monohydrate is dehydrated, and then the resultant dehydrated 4-chlorobenzenesulfonic acid is returned to the dehydration-condensing step (1), to produce an additional amount of dichlorodiphenylsulfone.

In the dehydrating step, preferably the recovered 4-chlorobenzenesulfonic acid monohydrate is converted to a substantially completely anhydrous compound and then the anhydrous 4-chlorobenzenesulfonic acid is brought into contact with chlorobenzene in the dehydration-condensing step (1).

The dehydration of the recovered 4-chlorobenzenesulfonic acid can be effected by a conventional dehydration method, preferably an azeotropic distillation dehydration method in which an azeotropic agent for water is used. The azeotropic agent usable for this dehydration step comprises at least one compound selected from those usable as the above-mentioned organic medium which is not compatible with water. Particularly, chlorobenzene is preferably used as an azeotropic agent for this dehydration step, because it is unnecessary to remove the azeotropic agent contained in the dehydrated 4-chlorobenzenesulfonic acid, before returning the dehydrated 4-chlorobenzenesulfonic acid to the dehydration-condensing step (1). For example, when an azeotropic distillation is applied to the recovered 4-chlorobenzenesulfonic acid monohydrate by using chlorobenzene as an azeotropic agent, the dehydration of the monohydrate compound is easily effected as long as the temperature of the dehydration liquid mixture is equal to or higher than the boiling point of the azeotropic mixture. In this step, the temperature of the dehydration liquid mixture depends on the contents of chlorobenzene and 4-chlorobenzenesulfonic acid, and can be controlled in accordance with the molar ratio of chlorobenzene to 4-chlorobenzenesulfonic acid. Preferably, the molar ratio of chlorobenzene to 4-chlorobenzenesulfonic acid is in the range of from 5:1 to 1:1, more preferably from 3:1 to 1:1 in consideration of the next step, namely the dehydration-condensing step (1). When the azeotropic distillation is applied, the composition of the dehydration liquid mixture varies and the temperature of the mixture raises with the progress of the dehydration. Accordingly, when an additional chlorobenzene is added to the mixture, in an amount equal to that removed from the dehydration liquid mixture, to keep the composition of the dehydration liquid mixture constant, an undesirable side reaction, for example, a hydrolysis of 4-chlorobenzenesulfonic acid, is restricted.

The dehydrated 4-chlorobenzenesulfonic acid per se is returned in the state of a solution in chlorobenzene to the dehydration-condensing step (1), to produce an additional amount of dichlorodiphenylsulfone. The conditions and operations for the dehydration-condensing reaction are the same as mentioned above.

For example, a mixture of the recovered 4-chlorobenzenesulfonic acid monohydrate with chlorobenzene is placed in a pressure-resistant closed reactor equipped with a condenser and a water-separator, and heated at a temperature at which 4-chlorobenzenesulfonic acid monohydrate is dehydrated while allowing an azeotropic mixture consisting of chlorobenzene and the resulting water to be continuously evaporated; the resultant azeotropic mixture vapor is condensed in the condenser; the condensed liquid mixture is introduced into the water-separator and separated into chlorobenzene and water to recover chlorobenzene; the recovered chlorobenzene is returned into the pressure-resistant reactor; and the separated water is discharged to the outside of the reactor. The resultant mixture in the reactor is in the state of a substantially anhydrous liquid. Then, the anhydrous liquid mixture is further heated at a temperature at which 4-chlorobenzenesulfonic acid is dehydration-condensed with chlorobenzene to produce dichlorodiphenylsulfone.

The process of the present invention is advantageous in that the resultant dichlorodiphenylsulfone can be separated from non-reacted 4-chlorobenzenesulfonic acid at a high efficiency, and the recovered 4-chlorobenzenesulfonic acid can be re-used in the reaction with chlorobenzene to produce an additional amount of dichlorodiphenylsulfone.

### EXAMPLES

The present invention will be further explained by the following specific examples.

### Example 1

A mixture of 560.7g of 4-chlorobenzenesulfonic acid having a water content of 1.89% by weight and containing 10.6g (0.589 mole) of water with 339.2g (3.102 moles) of chlorobenzene was heated at a temperature of 135°C and maintained at this temperature level while allowing an azeotropic mixture consisting of chlorobenzene and water to be azeotropically distilled, to prepare a reaction mixture having a water content of 670 ppm. During the above-mentioned dehydration-condensing reaction procedure, an additional chlorobenzene was added in an amount substantially equal to that of the azeotropically distilled chlorobenzene, to the reaction mixture, to keep the composition of the liquid portion of the reaction mixture constant.

The resultant reaction mixture was admixed with 174.6g (1.551 moles) of anhydrous chlorobenzene to provide a starting reaction mixture in an amount of 1075.1g for producing dichlorodiphenylsulfone.

The starting reaction mixture had the following composition.
- Chlorobenzene: : 505.9g (4.493 moles)
- 4-chlorobenzenesulfonic acid: : 547.8g (2.844 moles)
- Dichlorodiphenylsulfone: : 19.5g (0.068 mole)
- Water: : 0.2g
- Sulfuric acid: : 1.7g

The starting reaction mixture was continuously fed to a reactor having an inside diameter of 24 mm and a height of 1200 mm, and filled by glass beads heated at a temperature of 250°C through a top inlet thereof at a feeding rate of 200 g/hr, and a resultant reaction product mixture in an amount of 1064.5g was withdrawn from the reactor through a bottom outlet thereof. The resultant reaction product mixture had the following composition.
- Chlorobenzene: : 464.3g (4.123 moles)
- 4-chlorobenzenesulfonic acid: : 473.4g (2.458 moles)
- Dichlorodiphenylsulfone: : 115.7g (0.403 mole)
- Water: : 4.7g (0.261 mole)
- Sulfuric acid: : 6.4g (0.065 mole)

The yield of dichlorodiphenylsulfone was 11.79% based on the molar amount of 4-chlorobenzenesulfonic acid.

Then, a portion of the reaction product mixture in an amount of 432.4g was admixed with 453g (4.023 moles) of chlorobenzene, and the admixture was heated while stirring to provide a uniform solution. The solution was cooled to room temperature. Water in an amount of 15.9g (0.883 mole) was added dropwise to the cooled solution while stirring the solution. The addition of water caused a significant heat generation and a precipitation of the resultant 4-chlorobenzenesulfonic acid monohydrate was started. The dropping rate of water was controlled to such an extent that the temperature of the liquid mixture did not exceed 60°C. After completion of the dropwise water addition, the liquid mixture was continuously stirred. A slurry having a high fluidity was obtained.

The slurry was filtered under suction to recover the 4-chlorobenzenesulfonic acid monohydrate crystals separated from a chlorobenzene liquid phase containing dichlorodiphenylsulfone.

The recovered crystals comprised 191.7g (0.995 mole) of non-reacted 4-chlorobenzenesulfonic acid, 17.9g (0.995 mole) of water and 0.04g (1.39 x 10⁻⁴ mole) of dichlorodiphenylsulfone.

The chlorobenzene liquid phase was distilled to collect dichlorodiphenylsulfone by removing chlorobenzene.

The collected dichlorodiphenylsulfone was in an amount of 47.02g (0.164 mole) and contained 0.5g (2.6 x 10⁻³ mole) of non-reacted 4-chlorobenzenesulfonic acid. By the above-mentioned procedures, the resultant dichlorodiphenylsulfone and the non-reacted 4-chlorobenzenesulfonic acid were separated from each other and recovered at a very high distribution and recovery.

### Example 2

A mixture of 781.1g of recovered 4-chlorobenzenesulfonic acid monohydrate having a water content of 8.00% by weight and containing 62.5g (3.472 moles) of water with 718.6g (3.731 moles) of 4-chlorobenzenesulfonic acid and 709.8g (6.304 moles) of chlorobenzene was prepared, and subjected to the azeotropic dehydration in the same manner as in Example 1 to provide 1435.8g of a starting reaction mixture having a water content of 570 ppm.

The starting reaction mixture had the following composition.
- Chlorobenzene: : 709.8g (6.304 moles)
- 4-Chlorobenzenesulfonic acid: : 718.6g (3.731 moles)
- Dichlorodiphenylsulfone: : 4.0g (0.014 mole)
- Water: : 0.9g (0.050 mole)
- Sulfuric acid: : 2.6g (0.027 mole)

The starting reaction mixture was subjected to the same dehydration-condensing procedure as in Example 1 at a temperature of 250°C. A resultant reaction product mixture in an amount of 1429.1g was withdrawn from the reactor. The resultant reaction product mixture had the following composition.
- Chlorobenzene: : 663.5g (5.893 moles)
- 4-chlorobenzenesulfonic acid: : 609.2g (3.163 moles)
- Dichlorodiphenylsulfone: : 139.8g (0.487 mole)
- Water: : 4.7g (0.261 mole)
- Sulfuric acid: : 11.9g (0.121 mole)

The yield of dichlorodiphenylsulfone was 12.8% based on the molar amount of 4-chlorobenzenesulfonic acid.

Then, the reaction product mixture was admixed with 670g (5.950 moles) of chlorobenzene, and the admixture was heated while stirring to provide a uniform solution. The solution was cooled to room temperature. Water in an amount of 52.2g (2.90 moles) was gradually added dropwise to the cooled solution while stirring the solution, to cause the resultant 4-chlorobenzenesulfonic acid monohydrate to precipitate in the same manner as in Example 1.

The resultant slurry was filtered under suction to recover the 4-chlorobenzenesulfonic acid monohydrate crystals separated from a chlorobenzene liquid phase containing dichlorodiphenylsulfone.

The recovered crystals in an amount of 664.4g comprised 607.3g (3.153 moles) of non-reacted 4-chlorobenzenesulfonic acid, 56.8g (3.156 moles) of water and 0.3g (1.04 x 10⁻³ mole) of dichlorodiphenylsulfone.

The chlorobenzene liquid phase was distilled to collect dichlorodiphenylsulfone by removing chlorobenzene.

The collected dichlorodiphenylsulfone was in an amount of 139.5g (0.486 mole) and contained 1.9g (0.010 mole) of non-reacted 4-chlorobenzenesulfonic acid. In the above-mentioned procedures, the resultant dichlorodiphenylsulfone was collected at a yield of 12.67% based on the molar amount of 4-chlorobenzenesulfonic acid. This yield was comparative to that of Example 1 in which fresh 4-chlorobenzenesulfonic acid was employed. From this result, it became clear that the non-reacted 4-chlorobenzenesulfonic acid recovered in the form of monohydrate thereof can be fully re-used.

### Example 3

A mixture of the same recovered 4-chlorobenzenesulfonic acid monohydrate as in Example 2 with 400g of chlorobenzene was subjected to the same azeotropic dehydration as in Example 1 to provide 1255.2g of a starting reaction mixture having a water content of 550 ppm.

The starting reaction mixture had the following composition.
- Chlorobenzene: : 622.6g (5.529 moles)
- 4-chlorobenzenesulfonic acid: : 604.9g (3.141 moles)
- Dichlorodiphenylsulfone: : 21.6g (0.075 mole)
- Water: : 0.7g (0.039 mole)
- Sulfuric acid: : 5.4g (0.055 mole)

The starting reaction mixture was subjected to the same dehydration-condensing procedure as in Example 1 at a temperature of 250°C, and a resultant reaction product mixture in an amount of 1252.7g was withdrawn from the reactor. The resultant reaction product mixture had the following composition.
- Chlorobenzene: : 584.0g (5.186 moles)
- 4-chlorobenzenesulfonic acid: : 512.7g (2.662 moles)
- Dichlorodiphenylsulfone: : 134.4g (0.468 mole)
- Water: : 7.8g (0.433 mole)
- Sulfuric acid: : 13.8g (0.141 mole)

The yield of dichlorodiphenylsulfone was 14.6% based on the molar amount of 4-chlorobenzenesulfonic acid.

Then, the reaction product mixture was admixed with 550g (4.884 moles) of chlorobenzene, and the admixture was heated while stirring to provide a uniform solution. The solution was cooled to room temperature. Water in an amount of 40.1g (2.230 moles) was gradually added dropwise to the cooled solution in the same manner as in Example 1.

The resultant slurry was filtered under suction to recover the precipitated 4-chlorobenzenesulfonic acid monohydrate crystals separated from a chlorobenzene liquid phase containing dichlorodiphenylsulfone.

The recovered crystals in an amount of 559.1g comprised 511.2g (2.654 moles) of non-reacted 4-chlorobenzenesulfonic acid, 47.8g (2.654 moles) of water and 0.1g (3.50 x 10⁻⁴ mole) of dichlorodiphenylsulfone.

The chlorobenzene liquid phase was distilled to collect dichlorodiphenylsulfone by removing chlorobenzene.

The collected dichlorodiphenylsulfone was in an amount of 184.3g (0.467 mole) and contained 1.5g (7.8 x 10⁻³ mole) of non-reacted 4-chlorobenzenesulfonic acid.

In view of the above-mentioned results, the yield of dichlorodiphenylsulfone in this example was comparative to that in Example 1 in which fresh 4-chlorobenzenesulfonic acid was employed and in Example 2 in which the recovered 4-chlorobenzenesulfonic acid was employed. From this fact, it became clear that the non-reacted 4-chlorobenzenesulfonic acid twice-recovered and re-generated from the monohydrate thereof can be fully re-utilized to produce dichlorodiphenylsulfone.

### Example 4

A mixture of 64.30g of a recovered 4-chlorobenzenesulfonic acid monohydrate having a water content of 8.65% by weight and containing 5.56g (0.309 mole) of water with 58.74g (0.305 mole) of 4-chlorobenzenesulfonic acid and 56.98g (0.500 mole) of chlorobenzene was subjected to the same azeotropic dehydration as in Example 2, to prepare 119.50g of a starting reaction mixture having a water content of 410 ppm.

The starting reaction mixture had the following composition.
- Chlorobenzene: : 58.23g (0.517 mole)
- 4-chlorobenzenesulfonic acid: : 58.36g (0.303 mole)
- Dichlorodiphenylsulfone: : 2.89g (0.010 mole)
- Water: : 0.05g (0.003 mole)

The starting reaction mixture was fed to a pressure-resistant reactor and subjected to a batchwise dehydration-condensing procedure at a temperature of 250°C under a pressure of 9 kg/cm²G and a resultant reaction product mixture was withdrawn from the reactor. The resultant reaction product mixture had the following composition.
- Chlorobenzene: : 53.93g (0.479 mole)
- 4-chlorobenzenesulfonic acid: : 51.04g (0.265 mole)
- Dichlorodiphenylsulfone: : 13.95g (0.048 mole)

The yield of dichlorodiphenylsulfone was 12.53% based on the molar amount of 4-chlorobenzenesulfonic acid.

It was confirmed that the dehydration-condensing reaction was effected in the batchwise reaction procedure.

### Example 5

A mixture of 71.2g (0.632 mole) of chlorobenzene with 59.7g (0.156 mole) of anhydrous 4-chlorobenzenesulfonic acid was placed in an autoclave made from titanium and equipped with an electromagnetic stirrer, and subjected to a dehydration-condensing procedure at a temperature of 220°C under a pressure of 3 kg/cm²G. In this reaction procedure, chlorobenzene was continuously fed into the autoclave at a feeding rate of 225 g/hr while continuously withdrawing a chlorobenzene gas at a withdrawing rate of 224 g/hr to discharge the resultant water as an azeotropic mixture with the chlorobenzene gas to the outside of the autoclave. The reaction was continued for 7 hours.

The resultant reaction product mixture had the following composition.
- Chlorobenzene: : 61.0g (0.542 mole)
- 4-chlorobenzenesulfonic acid: : 30.0g (0.156 mole)
- Dichlorodiphenylsulfone: : 43.6g (0.152 mole)

The yield of dichlorodiphenylsulfone was 49.0% based on the molar amount of 4-chlorobenzenesulfonic acid.

### Example 6

A mixture of 138.7g of chlorobenzene with 116.8g of 4-chlorobenzenesulfonic acid having a water content of 0.7% by weight and containing 0.8g (0.044 mole) of water and 116.0g (0.602 mole) of 4-chlorobenzenesulfonic acid was placed in the same autoclave as in Example 5, and subjected to a dehydration-condensing procedure at a temperature of 230°C under a pressure of 4 kg/cm²G. In this reaction procedure, chlorobenzene was continuously fed into the autoclave at a feeding rate of 222 g/hr while continuously withdrawing a chlorobenzene gas at a withdrawing rate of 214.8 g/hr to discharge the resultant water as an azeotropic mixture with the chlorobenzene gas to the outside of the autoclave. The reaction was continued for 5 hours.

The resultant reaction product mixture had the following composition.
- Chlorobenzene: : 144.7g (1.285 moles)
- 4-chlorobenzenesulfonic acid: : 64.7g (0.336 mole)
- Dichlorodiphenylsulfone: : 76.5g (0.266 mole)

The yield of dichlorodiphenylsulfone was 44.2% based on the molar amount of 4-chlorobenzenesulfonic acid.

### Example 7

The reaction product mixture obtained in Example 5 was mixed with the reaction product mixture of Example 6, the mixture was subjected to the same procedure for recovering the non-reacted 4-chlorobenzenesulfonic acid as in Example 1. The recovered 4-chlorobenzenesulfonic acid monohydrate was dehydrated by the azeotropic dehydration procedure using chlorobenzene as an azeotropic agent. The resultant starting reaction mixture in an amount of 207.3g had the following composition.
- Chlorobenzene: : 112.6g (1.000 moles)
- 4-chlorobenzenesulfonic acid: : 94.4g (0.490 moles)
- Dichlorodiphenylsulfone: : 0.2g (7 x 10⁻⁴ mole)
- Water: : 0.1g (5.5 x 10⁻³ mole)

The starting reaction mixture was placed in the same autoclave as in Example 5, and subjected to a dehydration-condensing procedure at a temperature of 230°C under a pressure of 4 kg/cm²G for 5 hours. In this reaction procedure, chlorobenzene was continuously fed into the autoclave at a feeding rate of 230 g/hr while continuously withdrawing a chlorobenzene gas at a withdrawing rate of 225 g/hr to discharge the resultant water as an azeotropic mixture with the chlorobenzene gas to the outside of the autoclave.

The resultant reaction product mixture had the following composition.
- Chlorobenzene: : 112.5g (0.999 mole)
- 4-chlorobenzenesulfonic acid: : 51.4g (0.267 mole)
- Dichlorodiphenylsulfone: : 64.3g (0.224 mole)

The yield of dichlorodiphenylsulfone was 45.5% based on the molar amount of 4-chlorobenzenesulfonic acid.

### Comparative Example 1

The same starting reaction mixture as in Example 1 in an amount of 150.0g was mixed with 60g (0.533 mole) of chlorobenzene. To this mixture, 15.56g (0.864 mole) of water, which amount corresponded to 2.6 times the molar amount of 4-chlorobenzenesulfonic acid contained in the mixture, was added dropwise. After the heat generation was completed, the mixture was cooled to room temperature. The resultant 4-chlorobenzenesulfonic acid hydrate was precipitated in the state of a paste. The precipitated 4-chlorobenzenesulfonic acid hydrate was recovered by filtration under suction. The yield of the recovered hydrate compound was 13.34% in terms of anhydrous 4-chlorobenzenesulfonic acid. The chlorobenzene filtrate contained the remaining 4-chlorobenzenesulfonic acid hydrate in the state of a liquid.

## Claims

1. A process for producing dichlorodiphenylsulfone comprising the steps of:
(1) dehydration-condensing chlorobenzene with 4-chlorobenzenesulfonic acid while removing water generated as a by-product from the reaction mixture, to prepare dichlorodiphenylsulfone;
(2) adding water to the resultant reaction product mixture in a molar ratio of a non-reacted fraction of 4-chlorobenzenesulfonic acid to water of from 1:0.8 to 1:2, to convert the non-reacted 4-chlorobenzenesulfonic acid to a monohydrate thereof and to allow the resultant 4-chlorobenzenesulfonic acid monohydrate to precipitate from the reaction product mixture;
(3) recovering the precipitated 4-chlorobenzenesulfonic acid monohydrate from the reaction product mixture, to leave a residual reaction product solution; and
(4) collecting the reaction product consisting of dichlorodiphenylsulfone from the residual reaction product solution.

2. The process as claimed in claim 1, wherein the recovered 4-chlorobenzenesulfonic acid monohydrate is dehydrated and then the resultant dehydrated 4-chlorobenzenesulfonic acid is returned to the dehydration-condensing step (1), to produce an additional amount of dichlorodiphenylsulfone.

3. The process as claimed in claim 1, wherein the dehydration-condensing step (1) is carried out at a temperature of from 200°C to 290°C.

4. The process as claimed in claim 1, wherein the removal of water is continuously carried out by an azeotropic distillation of an azeotropic mixture of water with chlorobenzene.

5. The process as claimed in claim 1, wherein in the water-adding step (2), the reaction product mixture is in the state of a solution in which the reaction product consisting of dichlorodiphenylsulfone is completely dissolved in an organic medium.

6. The process as claimed in claim 5, wherein the organic medium for the solution of the reaction product mixture comprises a member selected from the group consisting of chlorobenzene, benzene, toluene, xylene, pseudocumene, tetrahydronaphthalene, dichloromethane, and 1,2-dichloroethane, and mixtures of two or more of the above-mentioned compounds.

7. The process as claimed in claim 1, wherein the recovering step (3) of the precipitated 4-chlorobenzenesulfonic acid monohydrate is carried out at a temperature lower than 101°C.

8. The process as claimed in claim 1, wherein in the collecting step (4), the resultant dichlorodiphenylsulfone is collected as a distillation residue of the residual reaction product solution.

9. The process as claimed in claim 2, wherein the dehydration of the recovered 4-chlorobenzenesulfonic acid monohydrate is carried out by mixing the recovered 4-chlorobenzenesulfonic acid with an azeotropic agent capable of forming together with water an azeotropic mixture, and then subjecting the resultant mixture to an azeotropic distillation to remove water together with the azeotropic agent.

10. The process as claimed in claim 9, wherein the azeotropic agent consists of chlorobenzene.

11. The process as claimed in claim 10, wherein chlorobenzene is present in a molar ratio to 4-chlorobenzene sulfonic acid of 5:1 to 1:1.

12. The process as claimed in claim 2 or 9, wherein the dehydrated 4-chlorobenzenesulfonic acid is returned in the state of a solution in chlorobenzene to the dehydration-condensing step (1).

13. The process as claimed in claim 2, wherein a mixture of the recovered 4-chlorobenzenesulfonic acid monohydrate with chlorobenzene is heated in a pressure-resistant closed reactor equipped with a condenser and a water separator to dehydrate 4-chlorobenzenesulfonic acid monohydrate and allow an azeotropic mixture consisting of chlorobenzene and the resulting water to be continuously evaporated; the resultant azeotropic mixture vapor is condensed in the condenser; the resultant condensed liquid mixture is introduced into the water separator to recover chlorobenzene; and the recovered chlorobenzene is returned into the reactor.
